(19) Europäisches Patentamt
European Patent Office
Office européen des brevets

(11) Veröffentlichungsnummer: 0 134 529
B1

(12) EUROPÄISCHE PATENTSCHRIFT

(45) Veröffentlichungstag der Patentschrifft: 21.03.90

(51) Int. Cl.[5]: C 07 J 53/00, A 61 K 31/585 // C07J31/00

(21) Anmeldenummer: 84108845.3

(22) Anmeldetag: 26.07.84

(54) **7-alpha-Substituierte 3-Oxo-17alpha-pregn-4-en-21.17-carbolactone, Verfahren zu ihrer Herstellung und diese enthaltende pharmazeutische Präparate.**

(30) Priorität: 17.08.83 DE 3330086
17.08.83 DE 3330084
17.08.83 DE 3330085

(43) Veröffentlichungstag der Anmeldung: 20.03.85 Patentblatt 85/12

(45) Bekanntmachung des Hinweises auf die Patenterteilung: 21.03.90 Patentblatt 90/12

(84) Bennante Vertragsstaaten: AT BE CH DE FR GB IT LI LU NL SE

(56) Entgegenhaltungen:
EP-A-0 061 418
EP-A-0 099 854
EP-A-0 115 273
US-A-3 013 012
US-A-3 509 136
US-A-3 787 396

(73) Patentinhaber: SCHERING AKTIENGESELLSCHAFT
Berlin und Bergkamen
Müllerstrasse 170/178 Postfach 65 03 11
D-1000 Berlin 65 (DE)

(72) Erfinder: Nickisch, Klaus, Dr.
Alt-Lichtenrade 11
D-1000 Berlin 49 (DE)
Erfinder: Laurent, Henry, Dr.
Glambecker Weg 21
D-1000 Berlin 28 (DE)
Erfinder: Esperling, Peter
Furkastrasse 15C
D-1000 Berlin 42 (DE)
Erfinder: Bittler, Dieter
Bölkauer Pfad 11
D-1000 Berlin 27 (DE)
Erfinder: Wiechert, Rudolf, Prof.Dr.
Petzower Strasse 8A
D-1000 Berlin 39 (DE)
Erfinder: Losert, Wolfgang, Prof.Dr.
Landshuter Strasse 22
D-1000 Berlin 30 (DE)

EP 0 134 529 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft neue 7α-substituierte 3-Oxo-17α-pregn-4-en-21,17-carbolactone, Verfahren zu ihrer Herstellung diese enthaltende pharmazeutische Präparate.

Im US-Patent 3 509 136 werden 3-[17β-Hydroxy-7α-(niederalkyl)-thio-3-oxo-an-drost-4-en-17α-yl]-propionsäure-γ-lactone genannt, die im Gegensatz zu den 7α-Alkylthio-3-oxo-17α-pregn-4-en-21,17-carbolactonen dieser Erfindung keine Methylengruppen in 1,2-und/oder 15,16-Stellung tragen.

Die Erfindung betrifft 7α-substituierte 3-Oxo-17α-pregn-4-en-21,17-carbolactone der allgemeinen Formel I

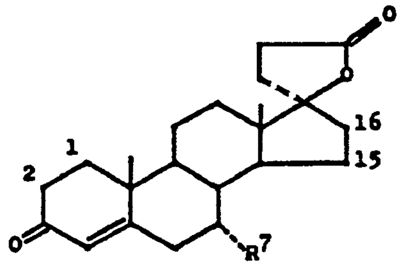

(I),

worin 2 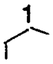 1

eine CC-Einfach- oder CC-Doppelbindung oder die Gruppe 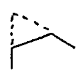

$R^7$ die Gruppe SR', wobei R' einen geradkettigen oder verzweigten, gegebenenfalls durch eine Hydroxygruppe substituierten Alkylrest von 1 – 6 Kohlenstoffatomen, und

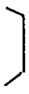 16 eine CC-Einfachbindung wenn 2 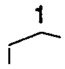 1 die Gruppe 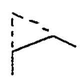 bedeutet, oder

15 die Gruppe 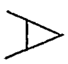 oder 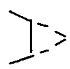

bedeuten.

R' in Formel I in der Bedeutung eines geradkettigen oder verzweigten gegebenenfalls durch eine Hydroxygruppe substituierten Alkylrestes von 1 bis 6 Kohlenstoffatomen stellt zum Beispiel eine Methyl-, Ethyl-, Propyl-, Isopropyl-, Butyl-, Hydroxyethyl-, 1- oder 2-Hydroxypropylgruppe dar.

Die neuen Verbindungen der allgemeinen Formel I haben die Eigenschaften, die Wirkung von Aldosteron oder Desoxycorticosteron auf die Natrium- und Kaliumsalzausscheidung aufzuheben bzw. umzukehren. Die erfindungsgemäßen Verbindungen sind damit geeignet zur Behandlung von bestimmten Formen der Hypertonie, von Ödemen, zum Beispiel bei Herzinsuffizienz, bei Leberzirrhose und nephrotischem Syndrom, des primären und sekundären Aldosteronismus und anderer durch Aldosteron bedingter endokrinologischer Störungen. Sie können außerdem als Diuretika eingesetzt werden.

Die neuen Wirkstoffe besitzen gegenüber dem handelsüblichen Spironolacton und seinen Metaboliten, die in 7α-Stellung statt der Acetylthio- eine Mercapto- oder Methylthiogruppe enthalten (Steroids 20 (1972) 41), den Vorteil der höheren Aktivität und der längeren Wirkungsdauer. Sie zeigen außerdem eine verminderte antiandrogene und gestagene Nebenwirkung.

Die erfindungsgemäßen Verbindungen, die wenigstens eine 1α,2α- oder 15α,16α-Methylengruppe enthalten, erweisen sich im Testmodell von Hollmann (Naunyn-Schmiedebergs Arch. Exp. Path. Pharmak. 247 (1964) 419) dem bekannten Spironolacton in ihrer Antialdosteronwirkung überraschenderweise überlegen und zeichnen sich gegenüber Spironolacton und den entsprechenden Verbindungen der deutschen Offenlegungsschrift 3 111 951, die keine 1α,2α- oder 15α,16α-Methylengruppe enthalten, durch eine geringere antiandrogene Nebenwirkung aus.

Die antiandrogene Wirkung wird bei Verbindungen festgestellt, die selber keine androgene Wirkung besitzen, aber durch ihre hohe Bindungsaffinität das körpereigene Androgen vom Rezeptor ganz oder teilweise verdrängen, wie das in gewissem Maß bei Spironolacton beobachtet wird. Es hat sich gezeigt, daß die neuen Verbindungen der allgemeinen Formel I eine geringere Affinität zum Androgenrezeptor besitzen als Spironolacton.

Im Gegensatz zum Spironolacton binden die erfindungsgemäßen Verbindungen nicht mehr an den Androgen- und Gestagenrezeptor. Das bedeutet, daß die neuen Verbindungen frei von antiandrogenen und gestagenen Nebenwirkungen sind.

Diese neuen Verbindungen der allgemeinen Formel I sind somit geeignet zur Behandlung von Ödemen, zum Beispiel bei schwerer Herzinsuffizienz, bei Leberzirrhose oder nephrotischem Syndrom sowie von Krankheitszuständen, an denen ein primärer oder sekundärer Hyperaldosteronismus beteiligt ist.

Die folgenden Verbindungen haben sich als besonders pharmakologisch wertvoll erwiesen:

1. 7α-Methylthio-1α,2α-methylen-3-oxo-17α-pregn-4-en-21,17-carbolacton,
2. 7α-Ethylthio-1α,2α-methylen-3-oxo-17α-pregn-4-en-21,17-carbolacton,

3. 7α-Propylthio-1α,2α-methylen-3-oxo-17α-pregn-4-en-21,17-carbolacton,
4. 7α-(2-Propylthio)-1α,2α-methylen-3-oxo-17α-pregn-4-en-21,17-carbolacton,
5. 7α-Butylthio-1α,2α-methylen-3-oxo-17α-pregn-4-en-21,17-carbolacton,
6. 7α-(2-Hydroxyethylthio)-1α,2α-methylen-3-oxo-17α-pregn-4-en-21,17-carbolacton,
7. 7α-Methylthio-15β,16β-methylen-3-oxo-17α-pregn-4-en-21,17-carbolacton,
8. 7α-Ethylthio-15β,16β-methylen-3-oxo-17α-pregn-4-en-21,17-carbolacton,
9. 7α-Propylthio-15β,16β-methylen-3-oxo-17α-pregn-4-en-21,17-carbolacton,
10. 7α-Buthylthio-15β,16β-methylen-3-oxo-17α-pregn-4-en-21,17-carbolacton,
11. 7α-Methylthio-15β,16β-methylen-3-oxo-17α-pregna-1,4-dien-21,17-carbolacton,
12. 7α-(2-Hydroxyethylthio)-15β,16β-methylen-3-oxo-17α-pregn-4-en-21,17-carbolacton,
13. 7α-(2-Hydroxyethylthio)-15β,16β-methylen-3-oxo-17α-pregna-1,4-dien-21,17-carbolacton,
14. 7α-(2-Hydroxypropylthio)-15β,16β-methylen-3-oxo-17α-pregn-4-en-21,17-carbolacton,
15. 7α-(2-Hydroxyethylthio)-15α,16α-methylen-3-oxo-17α-pregn-4-en-21,17-carbolacton,
16. 7α-(2-Hydroxyethylthio)-1α,2α;15β,16β-dimethylen-3-oxo-17α-pregn-4-en-21,17-carbolacton,
17. 7α-(2-Hydroxypropylthio)-1α,2α;15β,16β-dimethylen-3-oxo-17α-pregn-4-en-21,17-carbolacton,
18. 7α-Methylthio-1α,2α;15α,16α-dimethylen-3-oxo-17α-pregn-4-en-21,17-carbolacton,
19. 7α-Methylthio-15α,16α-methylen-3-oxo-17α-pregn-4-en-21,17-carbolacton,
20. 7α-(2-Hydroxyethylthio)-1α,2α;15α,16α-dimethylen-3-oxo-17α-pregn-4-en-21,17-carbolacton,
21, 7α-Methylthio-1α,2α;15β,16β-dimethylen-3-oxo-17α-pregn-4-en-21,17-carbolacton,
22. 7α-Ethylthio-1α,2α;15β,16β-dimethylen-3-oxo-17α-pregn-4-en-21,17-carbolacton,
23. 7α-(3-Hydroxypropylthio)-1α,2α;15β,16β-dimethylen-3-oxo-17α-pregn-4-en-21,17-carbolacton.

Die nachfolgende Tabelle zeigt die Antialdosteron-Wirkung an der Ratte sowie die Größe der Kompetitionsfaktoren (KF) als Maß für die Bindung am Androgen- bzw. am Gestagenrezeptor.

Hierbei bedeuten:

(+) Signifikant schlechtere Wirksamkeit der Prüfsubstanz
(++) Signifikant gleiche Wirksamkeit der Prüfsubstanz (bei 8 mg/Tier) im Vergleich zur Standardsubstanz
(+++) Signifikant bessere Wirksamkeit der Prüfsubstanz (bei 8 mg/Tier) im Vergleich zur Standardsubstanz.

**Tabelle**

| Verbindungs-Nr. bzw. Bezeichnung | Antialdosteronwirkung an der Ratte | Bindung an den Androgen-Rezeptor [KF] | Bindung an den Gestagen-Rezeptor [KF] |
|---|---|---|---|
| Spironolacton (SPL) | 1 | 8,9 | 21 |
| 2 | +++ | 9,2 | 36 |
| 7 | 0,95 x SPL | 87 | 75 |
| 11 | ~SPL | 82 | 47 |
| 12 | ++ | | |
| | 0,6 x SPL | ∞ | ∞ |
| 14 | 1,2 x SPL | ∞ | ∞ |
| 18 | +++ | 15 | 42 |
| 21 | 1,5 x SPL | 21 | ∞ |
| 22 | 1,5 x SPL | 13 | 32 |

Die Anwendung der neuen Wirkstoffe kann in der gleichen Weise erfolgen wie beim Spironolacton. Die Dosierung der Wirkstoffe liegt unterhalb der von Spironolacton. Aufgrund der zum Teil länger anhaltenden Wirkung brauchen die neuen Wirkstoffe in der Regel nur einmal am Tag appliziert zu werden.

Die Wirkstoffe können nach an sich bekannten Methoden der Galenik zu pharmazeutischen Präparaten, vorzugsweise für die enterale Applikation, verarbeitet werden. Für die enterale Applikation kommen insbesondere Tabletten, Dragees oder Kapseln infrage, die pro Dosiseinheit etwa 50 bis 200 mg Wirkstoff in einem inerten Trägermaterial enthalten.

Die Erfindung betrifft auch ein Verfahren zur Herstellung der erfindungsgemäßen Verbindungen der allgemeinen Formel I

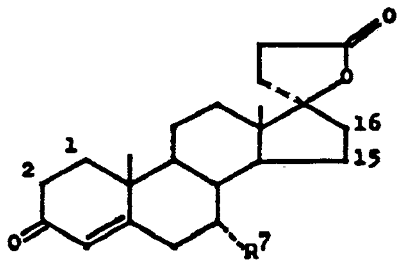

(I),

worin

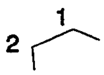 eine CC-Einfach- oder CC-Doppelbindung oder die Gruppe 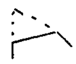

$R^7$ die Gruppe SR', wobei R' einen geradkettigen oder verzweigten, gegebenenfalls durch eine Hydroxygruppe substituierten Alkylrest von 1 – 6 Kohlenstoffatomen, und

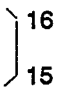 eine CC-Einfachbindung, wenn 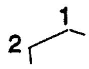 die Gruppe 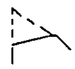

bedeutet,

oder die Gruppe 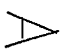 oder 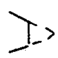

bedeuten, das

dadurch gekennzeichnet, daß man an eine $\Delta^6$-ungesättigte Verbindung der allgemeinen Formel III

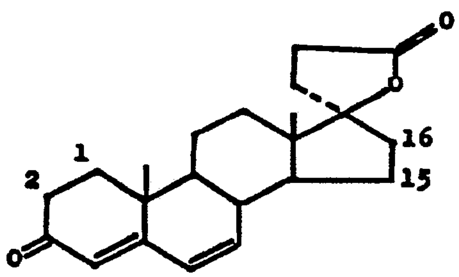

(III),

worin

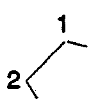 und 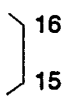 die in Formel I angegebene Bedeutung haben, einen Thioalkohol der Formel

R" – SH,

worin R" die in Formel I angegebene Bedeutung hat, addiert.

Zur Herstellung der 7α-Alkylthioverbindungen (R' = geradkettiger oder verzweigter gegebenenfalls durch eine Hydroxygruppe substituierter Alkylrest von 1 bis 6 Kohlenstoffatomen) wird an die $\Delta^6$-ungesättigte Verbindung der allgemeinen Formel III ein Thioalkohol der Formel R"-SH. worin R" die oben angegebene Bedeutung hat, addiert. Die Umsetzung wird in basischem Milieu in Gegenwart eines protischen Lösungsmittels wie Methanol oder Ethanol bei Temperaturen von 0 – 30°C vorgenommen. Als Basen sind insbesondere sekundäre oder tert.-Amine wie Diethylamin und Piperidin geeignet.

Die sich gegebenenfalls anschließende Einführung der $\Delta^1$-Doppelbindung kann in an sich bekannter Weise auf chemischem oder mikrobiologischem Wege erfolgen. Geeignete chemische Dehydrierungsmittel zur 1,2-Dehydrierung sind beispielsweise Selendioxid, 2,3-Dichlor-5,6-dicyanobenzochinon, Chloranil, Thalliumtriacetat oder Bleitetraacetat.

Geeignete Mikroorganismen für die 1,2-Dehydrierung sind beispielsweise Schizomyceten, insbesondere solche der Genera Arthrobacter, wie zum Beispiel A. simplex (ATCC 6946); Bacillus, wie zum Beispiel B. lentus (ATCC 13805) oder B. sphaericus (ATCC 7055); Pseudomonas, wie zum Beispiel P. aeruginosa (IFO 3505); Flavobacterium, wie zum Beispiel F. flavescens (IFO 3058) usw.

Die 1,2-Dehydrierung wird bevorzugt chemisch ausgeführt. Hierzu wird das 1,2-Dihydrosteroid in einem geeigneten Lösungsmittel mit dem Dehydrierungsmittel über längere Zeit auf 60 bis 120°C erhitzt. Geeignete Lösungsmittel sind beispielsweise Dioxan, Toluol, Benzol, Tetrahydrofuran, tert.-Butanol und Gemische dieser Lösungsmittel. Die Reaktion ist nach mehreren Stunden beendet. Es empfiehlt sich, die Umsetzung durch Dünnschichtchromatographie zu verfolgen. Das Reaktionsgemisch wird aufgearbeitet, wenn das Ausgangsmaterial umgesetzt ist.

Die zur Durchführung des erfindungsgemäßen Verfahrens benötigten Ausgangsverbindungen sind entweder bekannt (zum Beispiel aus US-Patentschrift 4 129 564) oder können nach einem der nachstehend beschriebenen Verfahren hergestellt werden.

**Herstellung der Ausgangsverbindungen**

A. 1α,2α-Methylen-3-oxo-17α-pregna-4,6-dien-21,17-carbolacton:

Eine Lösung von 15,7 g Trimethylsulfoxoniumiodid in 150 ml Dimethylsulfoxid wird unter Argon mit 2,84 g einer 55-prozentigen Suspension von Natriumhydrid in Mineralöl versetzt und so lange gerührt, bis eine klare Lösung entsteht. Anschließend werden 10 g 3-Oxo-17α-pregna-1,4,6-trien-21,17-carbolacton in fester Form hinzugegeben. Die Aufarbeitung erfolgt nach ca. 45 Minuten durch Eingießen in Eiswasser, das schwach mit Salzsäure angesäuert wurde. Das ausgefallene Produkt wird abfiltriert, mit Wasser gewaschen, getrocknet und danach an Kieselgel chromatographiert. Nach dem Umkristallisieren aus Aceton-Hexan erhält man 8,73 g 1α,2α-Methylen-3-oxo-17α-pregna-4,6-dien-21,17-carbolacton vom Schmelzpunkt 253,7°C. $[\alpha]_D$ = +176° (in Chloroform). UV: $\varepsilon_{282}$ = 21100 (in Methanol).

B. 1α,2α;15α,16α-Dimethylen-3-oxo-17α-pregna-4,6-dien-21,17-carbolacton:

1. 5,0 g 15α,16α,-Methylen-3-oxo-17α-pregna-4,6-dien-21,17-carbolacton werden in 50 ml Dioxan mit 5,0 g 2,3-Dichlor-5,6-dicyan-1,4-benzochinon 3 Stunden bei 100°C gerührt. Die Reaktionslösung wird dann abgekühlt, das ausgefallene Hydrochinon abgesaugt und mit Dioxan nachgewaschen. Das Filtrat wird im Vakuum weitgehend eingeengt. Der Rückstand wird in Diethylether aufgenommen, mit Natriumhydrogencarbonatlösung und Wasser gewaschen, getrocknet und eingedampft. Nach Chromatographie an Kieselgel werden 3,46 g 15α,16α-Methylen-3-oxo-17α-pregna-1,4,6-trien-21,17-carbolacton erhalten. UV: $\varepsilon_{222}$ = 11250, $\varepsilon_{254}$ = 9140, $\varepsilon_{299}$ = 11500 (in Methanol).

2. 8,8 g Trimethylsulfoxoniumiodid werden in 99 ml Dimethylsulfoxid mit 1,39 g Natriumhydrid, eine 55-prozentige Ölsuspension, bis zur Lösung des Hydrids gerührt. Dann werden unter Argon 2,8 g 15α,16α-Methylen-3-oxo-17α-pregna-1,4,6-trien-21,17-carbolacton zugegeben und 2 Stunden bei Raumtemperatur nachgerührt. Die Reaktionslösung wird in Eiswasser eingerührt, mit 2N Schwefelsäure schwach angesauert und der ausgefallene Niederschlag abfiltriert. Nach dem Auflösen in Methylenchlorid wird mit Wasser gewaschen, getrocknet und eingedampft. Der Rückstand wird an Kieselgel chromatographiert, und es werden 2,1 g 1α,2α;15α,16α-Dimethylen-3-oxo-17α-pregna-4,6-dien-21,17-carbolacton erhalten. UV. $\varepsilon_{281}$ = 19500 (in Methanol).

C. 1α,2α;15β,16β-Dimethylen-3-oxo-17α-pregna-4,6-dien-21,17-carbolacton:

1. Eine Lösung von 5,0 g 15β,16β-Methylen-3-oxo-17α-pregna-4,6-dien-21,17-carbolacton in 100 ml Toluol wird mit 5,0 g 2,3-Dichlor-5,6-dicyan-1,4-benzochinon versetzt und 20 Stunden auf 80°C erhitzt. Die abgekühlte Reaktionslösung wird über Kieselgel filtriert. Man wäscht mit Diethylether nach und erhält 4,54 g eines Rohprodukts, das nochmals an Kieselgel chromatographiert wird. Mit 9 – 12 % Aceton-Dichlormethan werden 3,88 g amorphes 15β,16β-Methylen-3-oxo-17α-pregna-1,4,6-trien-21,17-carbolacton eluiert. $[\alpha]_D$ = +10° (in Chloroform). UV: $\varepsilon_{225}$ = 10000. $\varepsilon_{255}$ = 8200, $\varepsilon_{301}$ = 10800 (in Methanol).

2. Eine Lösung von 6,09 g Trimethylsulfoxoniumiodid in 58 ml Dimethylsulfoxid wird unter Argon mit 1,1 g einer 55-prozentigen Suspension von Natriumhydrid in Mineralöl versetzt und 2 Stunden bei Raumtemperatur gerührt. Anschließend werden 3,88 g 15β,16β-Methylen-3-oxo-17α-pregna-1,4,6-trien-21,17-carbolacton in fester Form hinzugegeben. Das Reaktionsgemisch wird eine weitere Stunde gerührt und sodann in Eiswasser gegossen. Das ausgefällte Produkt wird abfiltriert, mit Wasser gewaschen und getrocknet und an Kieselgel chromatographiert. Mit 50 – 60 % Diethylether-Dichlormethan werden 3,49 g eluiert, die aus Dichlormethan-Diisopropylether umkristallisiert, 2,25 g 1α,2α;15β,16β-Dimethylen-3-oxo-17α-pregna-4,6-dien-21,17-carbolacton vom Schmelzpunkt 232,4°C ergeben. $[\alpha]_D$ = +198° (in Chloroform). UV: $\varepsilon_{282}$ = 20600 (in Methanol).

**Beispiel 1**

4,0 g 1α,2α-Methylen-3-oxo-17α-pregna-4,6-dien-21,17-carbolacton werden in 40 ml Methanol und 4 ml Piperidin gelöst. In die Lösung wird unter Eiskühlung aus einer Stahlflasche Methanthiol über einen Zeitraum von 30 Minuten eingeleitet. Anschließend läßt man 15 Stunden bei Raumtemperatur stehen und gießt die Mischung danach in Eiswasser. Das ausgefällte Produkt wird abfiltriert, mit Wasser gewaschen, getrocknet und an Kieselgel chromatographiert. Mit

8,8 – 10,3 % Aceton-Dichlormethan eluiert man 2,99 die aus Hexan-Dichlormethan-Diisopropylether umkristallisiert werden. Ausbeute 1,80 g 1α,2α-Methylen-7α-methylthio-3-oxo-17α-pregn-4-en-21,17α-carbolacton vom Schmelzpunkt 174,0°C. $[\alpha]_D$ = +137° (in Chloroform). UV: $\varepsilon_{236}$ = 13500 (in Methanol).

**Beispiel 2**

Eine Lösung von 3,0 g 1α,2α-Methylen-3-oxo-17α-pregna-4,6-dien-21,17-carbolacton in 30 ml Methanol und 3 ml Piperidin versetzt man mit 3 ml Ethanthiol. Das Reaktionsgemisch wird 15 Stunden bei Raumtemperatur gerührt und danach in Eiswasser gegossen. Das ausgefällte Produkt wird abfiltriert, mit Wasser gewaschen und in Dichlormethan aufgenommen. Die Lösung wird mit Wasser gewaschen, mit Natriumsulfat getrocknet und im Vakuum eingeengt. Der Rückstand wird an Kieselgel chromatographiert. Mit Essigester-Hexan (1 : 1) eluiert man 3,31 g die aus Dichlormethan-Diisopropylether umkristallisiert, 2,45 g 7α-Ethylthio-1α,2α-methylen-3-oxo-17α-pregn-4-en-21,17-carbolacton ergeben. Schmelzpunkt: 205,5°C. $[\alpha]_n$ = +115° (in Chloroform). UV: $\varepsilon_{236}$ = 13500 (in Methanol).

**Beispiel 3**

Unter den im Beispiel 2 angegebenen Bedingungen werden 3,0 g 1α,2α-Methylen-3-oxo-17α-pregna-4,6-dien-21,17-carbolacton mit 1-Propanthiol umgesetzt. Das Reaktionsprodukt wird entsprechend aufgearbeitet und gereinigt. Ausbeute: 1,57 g 1α,2α-Methylen-3-oxo-7α-propylthio-17α-pregn-4-en-21,17-carbolacton. Schmelzpunkt: 191,2°C. $[\alpha]_n$ = +103° (in Chloroform). UV: $\varepsilon_{236}$ = 13800 (in Methanol).

**Beispiel 4**

Unter den im Beispiel 2 angegebenen Bedingungen werden 1,5 g 1α,2α-Methylen-3-oxo-17α-pregna-4,6-dien-21,17-carbolacton in Gegenwart von 2-Propanthiol in 1α,2α-Methylen-3-oxo-7α-(2-propylthio)-17α-pregn-4-en-21,17-carbolacton überführt. Ausbeute: 891 mg. Schmelzpunkt: 239,4°C (aus Dichlormethan-Diisopropylether). $[\alpha]_n$ = +107° (in Chloroform). UV: $\varepsilon_{237}$ = 14000 (in Methanol).

**Beispiel 5**

Unter den im Beispiel 2 angegebenen Bedingungen wird 1,0 g 1α,2α-Methylen-3-oxo-17α-pregna-4,6-dien-21,17-carbolacton in Gegenwart von 1-Butanthiol in 7α-Butylthio-1α,2α-methylen-3-oxo-17α-pregn-4-en-21,17-carbolacton überführt. Ausbeute: 762 mg (aus Dichlormethan-Diisopropylether). $[\alpha]_n$ = +99° (in Chloroform). UV: $\varepsilon_{235}$ = 13900 (in Methanol).

**Beispiel 6**

Unter den im Beispiel 2 angegebenen Bedingungen werden 2,0 g 1α,2α-Methylen-3-oxo-17α-pregna-4,6-dien-21,17-carbolacton in Gegenwart von 2-Mercaptoethanol in 7α-(2-Hydroxyethylthio)-1α,2α-methylen-3-oxo-17α-pregn-4-en-21,17-carbolacton überführt. Ausbeute: 1,83 g (aus Dichlormethan-Diisopropylether). $[\alpha]_n$ = +106° (in Chloroform). UV: $\varepsilon_{236}$ = 13650 (in Methanol).

**Beispiel 7**

Unter den im Beispiel 1 angegebenen Bedingungen werden 1,2 g 15β,16β-Methylen-3-oxo-17α-pregna-4,6-dien-21,17-carbolacton in 15β,16β-Methylen-7α-methylthio-3-oxo-17α-pregn-4-en-21,17-carbolacton überführt. Ausbeute: 1,08 g. Schmelzpunkt: 268,7°C (aus Diisopropylether). UV: $\varepsilon_{238}$ = 16000 (in Methanol).

**Beispiel 8**

Unter den im Beispiel 2 beschriebenen Bedingungen werden 500 mg 15β,16β-Methylen-3-oxo-17α-pregna-4,6-dien-21,17α-carbolacton in 70-Ethylthio-15β,16β-methylen-3-oxo-17β-pregn-4-en-21,17-carbolacton überführt. Ausbeute: 450 mg. Schmelzpunkt: 261,2°C (aus Diisopropylether). UV: $\varepsilon_{238}$ = 16400 (in Methanol).

**Beispiel 9**

Unter den Bedingungen des Beispiels 2 werden 500 mg 15β,16β-Methylen-3-oxo-17α-pregna-4,6-dien-21,17-carbolac-

ton mit 1-Propanthiol in 15β,16β-Methylen-3-oxo-7α-propylthio-17β-pregn-4-en-21,17-carbolacton überführt. Ausbeute: 360 mg. Schmelzpunkt: 259,6°C (aus Aceton). UV: $\varepsilon_{241}$ = 15300 (in Methanol).

**Beispiel 10**

Unter den im Beispiel 2 beschriebenen Bedingungen werden 500 mg 15β,16β-Methylen-3-oxo-17α-pregna-4,6-dien-21,17-carbolacton in Gegenwart von 1-Butanthiol in 7α-Butylthio-15β,16β-methylen-3-oxo-17α-pregn-4-en-21,17-carbolacton überführt. Ausbeute: 390 mg. Schmelzpunkt. 262,7°C (aus Aceton). UV: $\varepsilon_{241}$ = 15600 (in Methanol).

**Beispiel 11**

Eine Lösung von 600 mg 15β,16β-Methylen-7α-methylthio-3-oxo-17α-pregn-4-en-21,17-carbolacton in 12 ml Dioxan wird mit 660 mg 2,3-Dichlor-5,6-dicyan-1.4-benzochinon versetzt und 17 Stunden bei 100°C gerührt. Die Reaktionsmischung wird mit Diethylether verdünnt, mit Natriumhydrogencarbonatlösung und Wasser gewaschen, getrocknet und eingedampft, der Rückstand wird an Kieselgel chromatographiert. Nach dem Verreiben mit Diisopropylether erhält man 145 mg 15β,16β-Methylen-7α-methylthio-3-oxo-17α-pregna-1,4-dien-21,17-carbolacton. Schmelzpunkt: 265,3°C. UV: $\varepsilon_{244}$ = 15400 (in Methanol).

**Beispiel 12**

Unter den im Beispiel 2 angegebenen Bedingungen werden 500 mg 15β,16β-Methylen-3-oxo-17α-pregna-4,6-dien-21,17-carbolacton in Gegenwart von 2-Mercaptoethanol in 7α-(2-Hydroxyethylthio)-15β,16β-methylen-3-oxo-17α-pregn-4-en-21,17-carbolacton überführt. Ausbeute: 530 mg. Schmelzpunkt: 233,2°C (aus Diisopropylether). UV: $\varepsilon_{238}$ = 15400 (in Methanol).

**Beispiel 13**

Die Lösung von 920 mg 7α-(2-Hydroxyethylthio)-15β,16β-methylen-3-oxo-17α-pregn-4-en-21,17-carbolacton in 18,5 ml Dioxan wird mit 920 mg 2,3-Dichlor-5,6-dicyan-1,4-benzochinon versetzt und 16 Stunden bei 80°C gerührt. Die Aufarbeitung und die Isolierung des Reaktionsprodukts wird analog Beispiel 17 vorgenommen. Ausbeute: 185 mg 7α-(2-Hydroxyethylthio)-15β,16β-methylen-3-oxo-17α-pregna-1,4-dien-21,17-carbolacton vom Schmelzpunkt 250°C (aus Diisopropylether). UV: $\varepsilon_{243}$ = 14900 (in Methanol).

**Beispiel 14**

Unter den im Beispiel 2 angegebenen Bedingungen werden 500 mg 15α,16β-Methylen-3-oxo-17α-pregna-4,6-dien-21,17-carbolacton durch Umsetzung mit 1-Mercapto-propan-2-ol in 7α-(2-Hydroxypropylthio)-15β,16β-methylen-3-oxo-17α-pregn-4-en-21,17-carbolacton überführt. Ausbeute: 419 mg. Schmelzpunkt: 250,3°C (aus Aceton-Diisopropylether). UV: $\varepsilon_{238}$ = 16200 (in Methanol).

**Beispiel 15**

Unter den im Beispiel 2 beschriebenen Bedingungen werden 500 mg 15α,16α-Methylen-3-oxo-17α-pregna-4,6-dien-21,17-carbolacton mit 2-Mercaptoethanol umgesetzt. Ausbeute: 365 mg 7α-(2-Hydroxyethylthio)-15α,16α-methylen-3-oxo-17α-pregn-4-en-21,17-carbolacton vom Schmelzpunkt 213,8°C. UV: $\varepsilon_{240}$ = 16650 (in Methanol).

**Beispiel 16**

Unter den Bedingungen des Beispiels 2 werden 500 mg 1α,2α;15β,16β-Dimethylen-3-oxo-17α-pregna-4,6-dien-21,17-carbolacton mit 2-Mercaptoethanol umgesetzt. Ausbeute: 385 mg 7α-(2-Hydroxyethylthio)-1α,2α;15β,16β-dimethylen-3-oxo-17β-pregn-4-en-21,17-carbolacton. Schmelzpunkt: 235,2°C (aus Diisopropylether-Aceton). UV: $\varepsilon_{240}$ = 12700 (in Methanol).

**Beispiel 17**

Unter den im Beispiel 2 beschriebenen Bedingungen werden 500 mg 1α,2α;15β,16β-Dimethylen-3-oxo-17α-pregna-4,6-dien-21,17-carbolacton mit 1-Mercapto-propan-2-ol umgesetzt. Reaktionszeit: 3 Tage. Ausbeute: 345 mg 7α-(2-Hydrox-

ypropylthio)-1α,2α;15β,16β-dimethylen-3-oxo-17α-pregn-4-en-21,17-carbolacton. Schmelzpunkt: 242°C (aus Aceton-Diisopropylether). UV: $\varepsilon_{236}$ = 13900 (in Methanol).

**Beispiel 18**

750 mg 1α,2α;15α,16α-Dimethylen-3-oxo-17α-pregna-4,6-dien-21,17-carbolacton werden, wie im Beispiel 1 beschrieben, mit Methanthiol zur Reaktion gebracht. Ausbeute: 540 mg 7α-Methylthio-1α,2α;15α,16α-dimethylen-3-oxo-17α-pregn-4-en-21,17-carbolacton als zähes Öl. UV: $\varepsilon_{237}$ = 12900 (in Methanol).

**Beispiel 19**

Unter den Bedingungen des Beispiels 1 werden aus 400 mg 15α,16α-Methylen-3-oxo-17α-pregna-4,6-dien-21,17-carbolacton 190 mg 7α-Methylthio-15α,16α-methylen-3-oxo-17α-pregn-4-en-21,17-carbolacton vom Schmelzpunkt 197,6°C (aus Diisopropylether) erhalten. UV: $\varepsilon_{238}$ = 14800 (in Methanol).

**Beispiel 20**

500 mg 1α,2α;15α,16α-Dimethylen-3-oxo-17α-pregna-4,6-dien-21,17-carbolacton werden unter den Bedingungen des Beispiels 2 mit 2-Mercaptoethanol umgesetzt . Ausbeute: 440 mg 7α-(2-Hydroxyethylthio)-1α,2α;15α,16α-dimethylen-3-oxo-17α-pregn-4-en-21,17-carbolacton als Öl. UV: $\varepsilon_{234}$ = 11800 (in Methanol).

**Beispiel 21**

1,2 g 1α,2α;15β,16β-Dimethylen-3-oxo-17α-pregna-4,6-dien-21,17-carbolacton werden unter den Bedingungen des Beispiels 1 in 1α,2α;15β,16β-Dimethylen-7α-methylthio-3-oxo-17α-pregn-4-en-21,17-carbolacton überführt. Ausbeute: 875 mg. Schmelzpunkt: 121,7°C (aus Diethylether-Diisopropylether). $[\alpha]_D$ = +118°. (in Chloroform). UV: $\varepsilon_{237}$ = 13000 (in Methanol).

**Beispiel 22**

1,2 g 1α,2α;15β,16β-Dimethylen-3-oxo-17α-pregna-4,6-dien-21,17-carbolacton werden unter den Bedingungen des Beispiels 2 in 7α-Ethylthio-1α,2α;15β,16β-dimethylen-3-oxo-17α-pregn-4-en-21,17-carbolacton überführt. Ausbeute: 1.06 g (aus Dichlormethan-Diisopropylether). $[\alpha]_D$ = +109° (in Chloroform). UV: $\varepsilon_{236}$ = 13700 (in Methanol).

**Beispiel 23**

Unter den Bedingungen des Beispiels 2 werden 500 mg 1α,2α;15β,16β-Dimethylen-3-oxo-17α-pregna-4,6-dien-21,17-carbolacton mit 3-Mercapto-propan-1-ol umgesetzt. Man erhält 275 mg 7α-(3-Hydroxypropylthio)-1α,2α;15β,16β-dimethylen-3-oxo-17α-pregn-4-en-21,17-carbolacton vom Schmelzpunkt 241,7°C. UV: $\varepsilon_{236}$ = 14100 (in Methanol).

**Beispiel 24**

7α-Methylthio-15α,16α-methylen-3-oxo-17α-pregna-1,4-dien-21,17-carbolacton.

420 mg 7α-Methylthio-15α,16α-methylen-3-oxo-17α-pregn-4-en-21,17-carbolacton werden in Toluol gelöst, mit 2,3-Dichlor-5,6-dicyano-1,4-benzochinon versetzt und 20 Std. auf 80° erhitzt. Nach Abkühlen, Filtrieren, Einengen in Vakuum wird der Rückstand an Kieselgel chromatographiert. Elution mit Essigester ergibt 195 mg 7α-Methylthio-15α,16α-methylen-3-oxo-17α-pregna-1,4-dien-21,17-carbolacton. UV: $\varepsilon_{242}$ = 14500 (Methanol).

**Patentansprüche**

1. 7α-Substituierte 3-Oxo-17α-pregn-4-en-21,17-carbolactone der allgemeinen Formel I

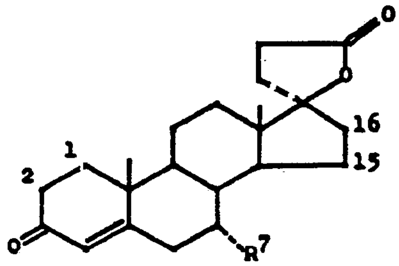

(I),

worin

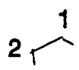 eine CC-Einfach- oder CC-Doppelbindung oder die Gruppe 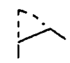

$R^7$ die Gruppe SR', wobei R' einen geradkettigen oder verzweigten, gegebenenfalls durch eine Hydroxygruppe substituierten Alkylrest von 1 – 6 Kohlenstoffatomen, und

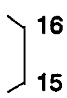 eine CC-Einfachbindung, wenn 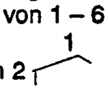 die Gruppe 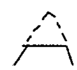 bedeutet, oder

die Gruppe 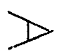 oder 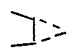

bedeuten.

2. 7α-Ethylthio-1α,2α-methylen-3-oxo-17α-pregn-4-en-21,17-carbolacton.

3. 7α-Methylthio-15β,16β-methylen-3-oxo-17α-pregn-4-en-21,17-carbolacton.

4. 7α-Methylthio-15β,16β-methylen-3-oxo-17α-pregna-1.4-dien-21,17-carbolacton.

5. 7α-(2-Hydroxyethylthio)-15β,16β-methylen-3-oxo-17α-pregn-4-dien-21,17-carbolacton.

6. 7α-(2-Hydroxypropylthio)-15β,16β-methylen-3-oxo-17α-pregn-4-en-21,17-carbolacton.

7. 7α-Methylthio-1α,2α;15β,16β-dimethylen-3-oxo-17α-pregn-4-en-21,17-carbolacton.

8. 7α-Ethylthio-1α,2α;15β,16β-dimethylen-3-oxo-17α-pregn-4-en-21,17-carbolacton.

9. 7α-Methylthio-1α,2α;15α,16α-dimethylen-3-oxo-17α-pregn-4-en-21,17-carbolacton.

10. Pharmazeutische Präparate, gekennzeichnet durch einen Gehalt an Verbindungen gemäß den Ansprüchen 1 bis 9.

11. Verfahren zur Herstellung von 7α-substituierten 3-Oxo-17α-pregn-4-en-21,17-carbolactonen der allgemeinen Formel I

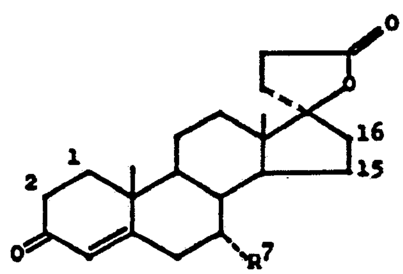

(I),

worin

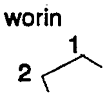 eine CC-Einfach- oder CC-Doppelbindung oder die Gruppe 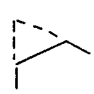

$R^7$ die Gruppe SR', wobei R' einen geradkettigen oder verzweigten, gegebenenfalls durch eine Hydroxygruppe substituierten Alkylrest von 1 – 6 Kohlenstoffatomen, und

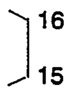 eine CC-Einfachbindung wenn 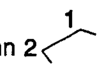 die Gruppe 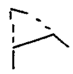 bedeutet, oder die Gruppe 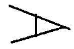 oder 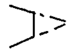

bedeuten,

dadurch gekennzeichnet, daß man an eine $\Delta^6$-ungesättigte Verbindung der allgemeinen Formel III

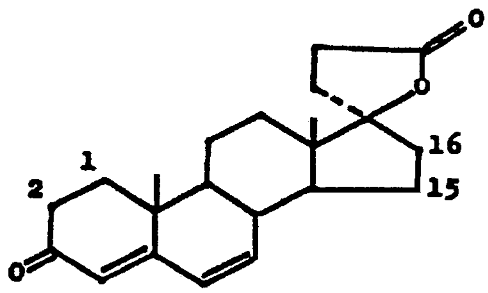 (III),

worin

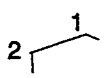 und 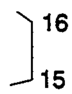 die in Formel I angegebene Bedeutung haben, einen Thioalkohol der Formel

R" – SH,

worin R" die in Formel I angegebene Bedeutung hat, addiert.

**Claims**

1. 7α-substituted 3-oxo-17α-pregn-4-ene-21,17-carbolactones of the general formula I

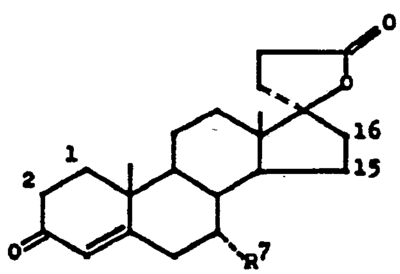 (I)

wherein

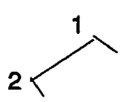 is a CC single or CC double bond or the group 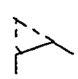

$R^7$ is the group SR' wherein R' is a straight-chain or branched alkyl radical having from 1 to 6 carbon atoms that is optionally substituted by a hydroxy group, and

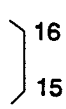 is a CC single bond when 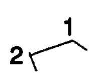 represents the group 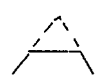, or is the group 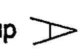 or 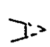.

2. 7α-Ethylthio-1α,2α-methylene-3-oxo-17α-pregn-4-ene-21,17-carbolactone.

3. 7α-Methylthio-15β,16β-methylene-3-oxo-17α-pregn-4-ene-21,17-carbolactone.

4. 7α-Methylthio-15β,16β-methylene-3-oxo-17α-pregna-1,4-diene-21,17-carbolactone.

5. 7α-(2-Hydroxyethylthio)-15β,16β-methylene-3-oxo-17α-pregn-4-ene-21,17-carbolactone.

6. 7α-(2-Hydroxypropylthio)-15β,16β-methylene-3-oxo-17α-pregn-4-ene-21,17-carbolactone.

7. 7α-Methylthio-1α,2α;15β,16β-dimethylene-3-oxo-17α-pregn-4-ene-21,17-carbolactone.

8. 7α-Ethylthio-1α,2α;15β,16β-dimethylene-3-oxo-17α-pregn-4-ene-21, 17-carbolactone.

9. 7α-Methylthio-1α,2α;15α,16α-dimethylene-3-oxo-17α-pregn-4-ene-21,17-carbolactone.

10. Pharmaceutical preparations, characterised by a content of compounds according to claims 1 to 9.

11. Process for the preparation of 7α-substituted 3-oxo-17α-pregn-4-ene-21,17-carbolactones of the general formula I

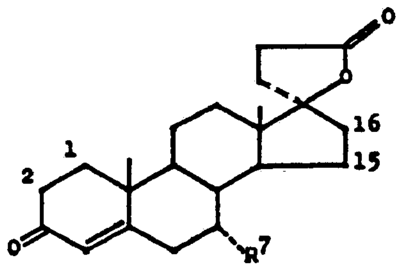

(I)

wherein

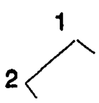 is a CC single or CC double bond or the group 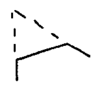

$R^7$ is the group SR' wherein R' is a straight-chain or branched alkyl radical having from 1 to 6 carbon atoms that is optionally substituted by a hydroxy group, and

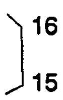 is a CC single bond when 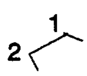 represents the group 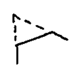,

or is the group 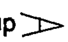 or 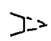,

characterised in that a thioalcohol of formula

R" – SH

wherein R" has the meaning given for formula I, is added to a $\Delta^6$-unsaturated compound of the general formula III

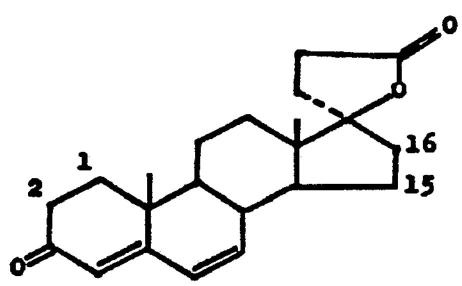

(III)

wherein

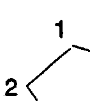 and 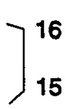 have the meanings given for formula I.

**Revendications**

1. 3-oxo-17α-pregn-4-ène-21,17-carbolactones substituées en position 7α de formule générale I

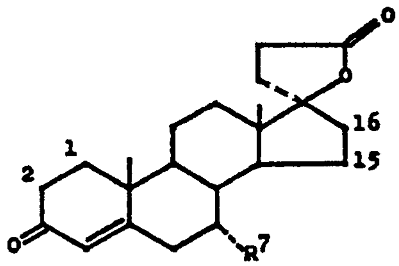

(I),

dans laquelle

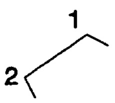 représente une liaison CC simple ou une double liaison CC, ou encore le groupe 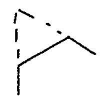

$R^7$ représente le groupe SR', où R' est un radical alkyle ayant de 1 à 6 atomes de carbone, à chaîne droite ou ramifiée éventuellement substitué par un groupe hydroxy, et

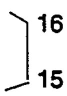 représente une simple liaison CC quand 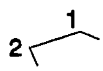 représente le groupe 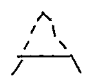, ou encore le groupe 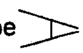 ou 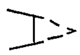.

2. 7α-méthylthio-1α,2α-méthylène-3-oxo-17α-pregn-4-ène-21,17-carbolactone.

3. 7α-méthylthio-15β,16β-méthylène-3-oxo-17α-pregn-4-ène-21,17-carbolactone.

4. 7α-méthylthio-15β,16β-méthylène-3-oxo-17α-pregn-4-ène-21,17-carbolactone.

5. 7α-(2-hydroxyéthylthio)-15β,16β-méthylène-3-oxo-17α-pregn-4-ène-21,17-carbolactone.

6. 7α-(2-hydroxypropylthio)-15β,16β-méthylène-3-oxo-17α-pregn-4-ène-21,17-carbolactone.

7. 7α-méthylthio-1α,2α;15β,16β-diméthylène-3-oxo-17α-pregn-4-ène-21,17-carbolactone.

8. 7α-éthylthio-1α,2α;15β,16β-diméthylène-3-oxo-17α-pregn-4-ène-21,17-carbolactone.

9. 7α-méthylthio-1α,2α;15α,16α-diméthylène-3-oxo-17α-pregn-4-ène-21,17-carbolactone.

10. Préparations pharmaceutiques, caractérisées en ce qu'elles contiennent des composés selon les revendications 1 à 9.

11. Procédé pour la préparation de 3-oxo-17α-pregn-4-ène-21,27-carbolactones substituées en position 7α de formule générale I

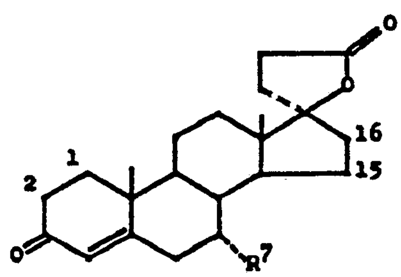

(I),

dans laquelle

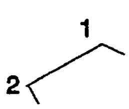 représente une liaison CC simple ou une double liaison CC, ou encore le groupe 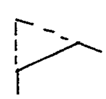

$R^7$ représente le groupe SR', où R' est un radical alkyle ayant de 1 à 6 atomes de carbone, à chaîne droite ou ramifiée, éventuellement substitué par un groupe hydroxy, et

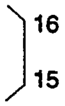 représente une simple liaison CC quand 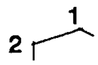 représente le groupe 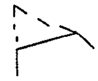 ,

ou encore le groupe 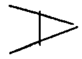 ou 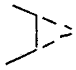

caractérisé en ce qu'on fixe par addition à un composé $\Delta^6$-insaturé de formule générale III

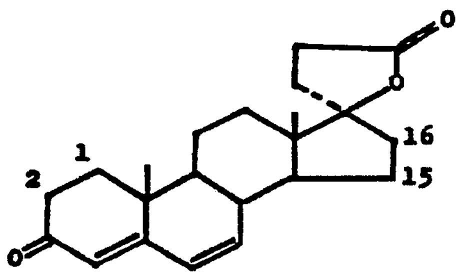

(III),

dans laquelle

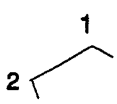 et 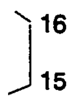

ont les significations données dans la formule I, un thioalcool de formule

R" – SH,

où R" a les significations données pour la formule I.